(19) Europäisches Patentamt
European Patent Office
Office européen des brevets

(11) **EP 3 199 950 A1**

(12) **EUROPEAN PATENT APPLICATION**
published in accordance with Art. 153(4) EPC

(43) Date of publication:
**02.08.2017 Bulletin 2017/31**

(21) Application number: **16755594.5**

(22) Date of filing: **25.02.2016**

(51) Int Cl.:
***G01N 33/574*** *(2006.01)*

(86) International application number:
**PCT/JP2016/055541**

(87) International publication number:
**WO 2016/136850 (01.09.2016 Gazette 2016/35)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **27.02.2015 JP 2015037717**

(71) Applicant: **J-Oil Mills, Inc.**
**Tokyo 104-0044 (JP)**

(72) Inventors:
- **KUSAMA, Ken**
  **Tokyo 104-0044 (JP)**
- **KOBAYASHI, Yuka**
  **Tokyo 104-0044 (JP)**
- **UENO, Yasushi**
  **Tokyo 104-0044 (JP)**

(74) Representative: **Gallego Jiménez, José Fernando**
**Ingenias Creaciones, Signos e Invenciones S.L.P.**
**Avda. Diagonal 421, 2**
**08008 Barcelona (ES)**

(54) **SIGNAL ENHANCER**

(57) [Problem] To provide a signal enhancer that enhances a signal based on reaction between an α1-6 fucose sugar chain and an α1-6 fucose specific lectin bound thereto, a method for using the same, and use of the same.

[Solution] A signal enhancer enhances a signal based on reaction between an α1-6 fucose sugar chain and an α1-6 fucose specific lectin bounded thereto, and is characterized by having, as an active ingredient, at least one selected from the group consisting of urea and thiourea. The final concentration of the urea is preferably 1-9 M and the final concentration of the thiourea is 0.1-1.5 M. Since the signal enhancer allows accurate measurement of an α1-6 fucose sugar chain, the signal enhancer is greatly useful in detection and determination of a disease related to an α1-6 fucose sugar chain and in academic study of an α1-6 fucose sugar chain.

Reaction value (S-N)
1.0
0.8
0.6
0.4
0.2
0.0
Urea 0M
Urea 5M
1000ng/ml fHP
1000ng/ml AFP-L3
5000ng/ml IgG
5000ng/ml TF
Example 1
Example 2
Example 3
Comp.Example 1

Fig.1

EP 3 199 950 A1

## Description

### Technical Field

The present invention relates to a signal enhancer, more specifically an enhancer for signals based on a reaction between an α1-6 fucose sugar chain and an α1-6-fucose-specific lectin.

### Background Art

A gene of an α1-6 fucose transferase (α1-6FucT) which transfers an α1-6 fucose (also referred to as core fucose) to N-acetylglucosamine at a reducing terminal of an N-linked sugar chain is known to be expressed in association with canceration of hepatic cells. Once hepatocarcinoma is caused, the fucose is transferred, through an α1-6 bond, to N-acetylglucosamine which is a sugar chain in a glycoprotein α-fetoprotein (AFP) having a molecular weight of about 70,000. Currently, the AFP to which the fucose is transferred through this α1-6 bond is used as a tumor marker for hepatocarcinoma. When a subject's serum is subjected to a lectin affinity electrophoresis using a Lentil lectin (LCA) having affinity for the fucose, three bands called AFP-L1, AFP-L2 and AFP-L3 emerge from the anode side. Although benign liver diseases such as hepatic cirrhosis mainly show lectin non-binding type AFP-L1 bands, lectin-binding type AFP-L3 bands increase when hepatocarcinoma is caused. When a ratio of AFP-L3 to the total AFP (AFP-L3 %) is determined, if its value exceeds 10%, hepatocarcinoma is suspected. If the ratio of the AFP to which fucose has been transferred through the α1-6 bond can be accurately recognized, accuracy of hepatocarcinoma diagnosis will also be further enhanced.

The α1-6 fucose is known to relate to, besides liver cancers, diseases such as pancreatic cancer and colon cancer.

Conventionally, as a lectin with affinity for α1-6 fucose, lentil lectin (LCA), pea lectin (PSA), Aleuria aurantia lectin (AAL), Narcissus pseudonarcissus lectin (NPA), Vicia faba lectin (VFA), Aspergillus oryzae lectin (AOL) and the like were known. However, the AAL and AOL also have affinity for α1-2 fucose, α1-3 fucose and the like, and the LCA, PSA and VFA also have affinity for an N-linked single and/or double sugar chains having no fucose α1-6. As such, these lectins also have an affinity for, besides the α1-6 fucose sugar chain, a glycolipid-based sugar chain having a fucose other than α1-6 bond, and a high-mannose sugar chain having no fucose.

The inventors have newly found lectins such as Pholiota squarrosa lectin (PhoSL) (Non-Patent Document 1), and Stropharia rugosoannulata lectin (SRL) (Patent Document 1) from natural mushrooms, as lectins that more specifically detect the α1-6 fucose sugar chain than the conventionally known lectin. Furthermore, chemical synthesis and recombinant production of these lectins have been also carried out (Patent Documents 4 and 5). In addition, it has also succeeded in detection of AFP-L3 (Patent Document 1), determination of a deterioration degree in colon cancer (Patent Document 2), and detection of pancreatic cancer (Patent Document 3), by using these lectins.

### Prior Art Documents

#### Patent Documents

Patent Document 1: JP Pat. No. 4514163 (Fucose α1→6-specific lectin)
Patent Document 2: JP Pat. No. 5263979 (Method for determining colon cancer)
Patent Document 3: JP Pat. No. 4900983 (Method for detecting pancreatic cancer)
Patent Document 4: JP 2011-148736 A (Peptide)
Patent Document 5: JP 2011-148735 A (Gene)


#### Non-Patent Documents


Non-Patent Document 1: Yuka Kobayashi et al., "A Novel Core Fucose-specific Lectin from the Mushroom Pholiota squarrosa", J. Biol. Chem, 2012, 287, p 33973-33982


### Summary of Invention

#### Problem to be solved

The α1-6 fucose-specific lectin discovered by the inventors enabled specific detection of an α1-6 fucose sugar

chain. If signals based on a reaction between the α1-6 fucose sugar chain and an α1-6 fucose-specific lectin can be enhanced, the α1-6 fucose sugar chain can be accurately detected. Thus, an object of the present invention is to provide a signal enhancer that enhances signals based on the reaction between the α1-6 fucose sugar chain and the α1-6 fucose-specific lectin binding thereto.

## Solution to Problem

As a result of earnest investigations for the above problems, the inventors found that the above problems could be solved according to the following inventions, and completed the present invention. That is, the present invention provides a signal enhancer which enhances signals based on a reaction between an α1-6 fucose sugar chain and an α1-6 fucose-specific lectin binding thereto, characterized in that it has, as an active ingredient, at least one selected from a group consisting of urea and thiourea. The term "α1-6 fucose sugar chain" herein means "a structure in which a fucose binds to N-acetylglucosamine at a reducing end of an N-type sugar chain through an α1-6 bond". In addition, the term "α1-6 fucose-specific lectin" herein means "lectin characterized in that (1) it has affinity for the α1-6 fucose sugar chain represented by a binding constant $1.0\times10^4$ $M^{-1}$ or more (at 25°C) and that (2) it does not substantially bind to a high mannose sugar chain containing no α1-6 fucose and/or a glycolipid sugar chain containing no α1-6 fucose".

JP 2010-145202 A discloses a method for enhancing an antigen-antibody reaction, in which antigen and antibody are reacted in coexistence of polyethylene glycol and urea. The reaction to which the signal enhancer of the present invention is applied is a reaction between an α1-6 fucose sugar chain and an α1-6 fucose-specific lectin binding thereto, but not an antigen-antibody reaction, and is clearly different from the invention of JP 2010-145202 A in that no polyethylene glycol is required.

The present invention also provides to a method for enhancing signals based on a reaction between an α1-6 fucose sugar chain and an α1-6 fucose-specific lectin binding thereto, which includes a step of reacting the sugar chain with the lectin in the presence of a signal enhancer having, as an active ingredient, at least one selected from a group consisting of urea and thiourea.

The present invention also provides a method for enhancing signals based on a reaction between an α1-6 fucose sugar chain and an α1-6 fucose-specific lectin binding thereto, which includes a step of washing the sugar chain with a detergent liquid which contains a signal enhancer having, as an active ingredient, at least one selected from a group consisting of urea and thiourea, and a step of reacting the sugar chain with the lectin.

The present invention also provides a kit for detecting an α1-6 fucose sugar chain, which comprises a signal enhancer having, as an active ingredient, at least one selected from a group consisting of urea and thiourea and an α1-6 fucose-specific lectin.

## Effects of Invention

The signal enhancer of the present invention essentially containing at least one selected from the group consisting of urea and thiourea (hereinafter referred to as urea etc.) can sensitize the reaction between the α1-6 fucose sugar chain and the α1-6 fucose-specific lectin binding thereto, and enhance the signals based on the reaction, as described in Examples set forth below. The urea etc. do not sensitize a reaction between a sugar chain having no α1-6 fucose sugar chain (e.g. transferrin) and an α1-6 fucose-specific lectin, as described in Comparative Example 1 set forth below. In addition, the urea etc. do not sensitize a reaction between the sugar chain having the α1-6 fucose sugar chain and a lectin having affinity for the α1-6 fucose but no specificity thereto (e.g. AAL), as described in Comparative Example 2. Although data are not shown, the urea etc. also do not sensitize a reaction between the sugar chain having the α1-6 fucose sugar chain and a lectin having no affinity for the α1-6 fucose (e.g. Ulex europaeus lectin 1 (UEA-1), Lotus corniculatus lectin (Lotus), jack bean lectin (Con A), etc.). Consequently, the signal enhancer of the present invention containing urea etc. is directed to the specific reaction between the α1-6 fucose sugar chain and the α1-6 fucose-specific lectin.

Since the signal enhancer of the present invention allows accurate measurement of an α1-6 fucose sugar chain, the signal enhancer is greatly useful in detection and determination of a disease related to an α1-6 fucose sugar chain and academic study of an α1-6 fucose sugar chain.

## Brief Description of Drawings

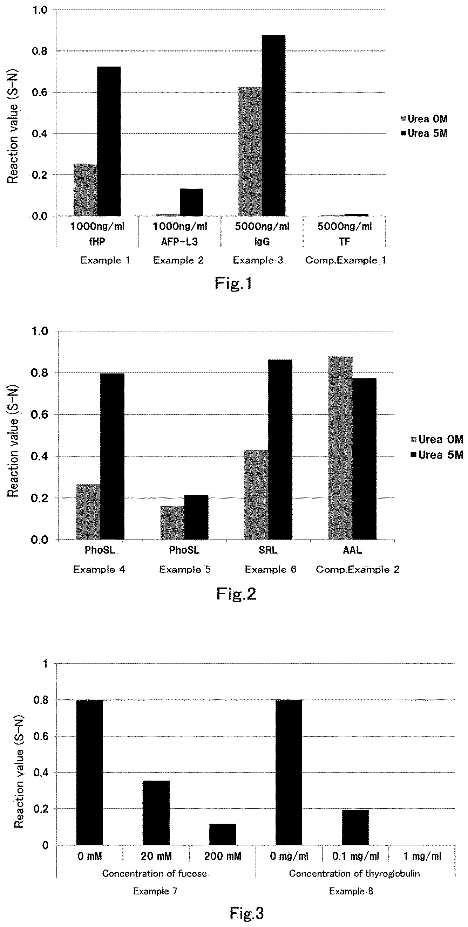

[Fig. 1] Figure 1 illustrates bar graphs showing the results of examining the reaction value (S - N) between an α1-6 fucose-specific lectin and various glycoproteins, in the presence and absence of urea. Of the bar graphs, the left-sided bars indicate the absence of urea and the right-sided bars indicate the presence of 5 M urea. The reactivity

of PhoSL to any of fHP, AFP-L3 and IgG having anal-6 fucose sugar chain is also enhanced in the presence of urea. On the other hand, the reactivity to TF not belonging to the α1-6 fucose sugar chain does not change even in the presence of urea.

[Fig. 2] Figure 2 illustrates bar graphs showing the results of examining the reaction value (S - N) between the α1-6 fucose-specific lectin (PhoSL, PhoSL peptide and SRL) or a lectin having affinity for the α1-6 fucose but no specificity thereto (AAL) and fHP, in the presence and absence of urea. Of the bar graphs, the left-sided bars indicate the absence of urea and the right-sided bars indicate the presence of 5 M urea. The reaction value (S - N) between all the α1-6 fucose-specific lectins and fHP increases in the presence of urea. On the other hand, the reaction value (S - N) between the AAL having affinity for the α1-6 fucose but no specificity thereto and fHP does not increase even in the presence of urea.

[Fig. 3] Figure 3 illustrates bar graphs made by examining the reaction between PhoSL and fHP in the presence of urea and fucose (Example 7) or in the presence of urea and thyroglobulin (Example 8). The reaction between PhoSL and fHP in the presence of urea is inhibited in a concentration-dependent manner by fucose or thyroglobulin. This indicates that urea is involved in the α1-6 fucose-specific binding between PhoSL and fHP.

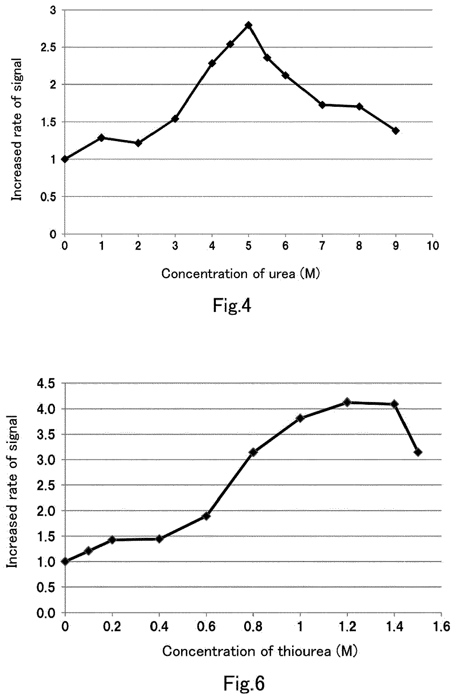

[Fig. 4] Figure 4 illustrates a graph of the reaction values (S - N) obtained by measuring the reaction between fHP and PhoSL while changing the concentration of the coexisting urea. Figure 4 shows that the signals are enhanced by 1.2 to 2.8 times with a urea concentration of 1 to 9 M.

[Fig. 5] Figure 5 illustrates a graph of the reaction values (S - N) obtained by measuring the reaction between fHP and PhoSL while changing the concentration of the coexisting thiourea. Figure 5 shows that the signals are enhanced by 1.2 to 4.1 times with a thiourea concentration of 0.1 to 1.5 M.

**Description of Embodiments**

The signal enhancer of the present invention is a signal enhancer that enhances signals based on a reaction between anal-6 fucose sugar chain and anal-6 fucose-specific lectin binding thereto, and characterized in that it has at least one selected from a group consisting of urea and thiourea.

The concentration of urea in the signal enhancer may be typically 1 to 9 M, and is preferably 2.5 to 9 M, more preferably 3 to 8 M, and particularly preferably 3.5 to 7 M. In addition, the concentration of thiourea in the signal enhancer may be typically 0.1 to 1.5 M, and is preferably 0.6 to 1.5 M, more preferably 0.8 to 1.5 M, and particularly preferably 1 to 1.4 M.

The urea etc. in the signal enhancer are preferably dissolved in a solvent for use. Examples of the solvent include: water; a saline such as phosphate buffered saline and Tris buffered saline; a buffer such as phosphate buffer and Tris buffer; a buffer in which a protein such as bovine serum albumin is dissolved, and its aqueous solution; and a buffer comprising a surfactant such as Tween-20, and its aqueous solution.

The pH of the signal enhancer is typically 4 to 10, and preferably 5 to 9.

The above-described α1-6 fucose sugar chain means a structure in which a fucose binds to an N-acetylglucosamine at a reducing terminal of an N-type sugar chain through an α1-6 bond, and as long as the α1-6 fucose sugar chain is an N-linked sugar chain, it encompasses all of free oligosaccharide chains, glycopeptides, glycoproteins, cells, etc. Also, it includes sugar chains prepared by chemically modifying oligosaccharide chains etc. such as labels. The N-linked sugar chain may be a high mannose type, a complex type, a hybrid type or the like. Furthermore, the sugar chain may be: a sugar chain prepared by partially decomposing the sugar chain with acid, hydrazine or the like in a chemical manner; and a sugar chain prepared by partially decomposing the sugar chain with simultaneous or stepwise use of any enzyme of sialidase, galactosidase, N-acetylhexosaminidase, fucosidase cleaving α1-2 fucose, α1-3 fucose and α1-4 fucose, and mannosidase. In addition, it may be a sugar chain prepared by adding a sugar such as glucose, a functional group such as an acetyl group, a sulfate group and a phosphate group and the like to the above-described sugar chain.

A representative example of the fucose α1-6 sugar chain is shown below.

[Formula 1]

Manα1 → 6 Manβ1 → 4GlcNAcβ1 → 4GlcNAc- (Fucα1 → 6)
Manα1 → 3

[wherein, Man means mannose, GlcNAc means N-acetylglucosamine, Fuc means fucose]

Particularly preferable specific examples for the α1-6 fucose sugar chain are tumor markers such as AFP-L3 and fucosylated haptoglobin.

The α1-6 fucose-specific lectin is characterized in that it more specifically binds to the α1-6 fucose sugar chain than the conventional lectin (e.g. AAL) having affinity for the α1-6 fucose sugar chain. This characteristic can be defined by (1) the lower limit of the binding constant for the α1-6 fucose sugar chain and (2) the upper limit of the binding constant for the high-mannose sugar chain containing no α1-6 fucose and/or the glycolipid sugar chain containing no α1-6 fucose. That is, the α1-6 fucose-specific lectin is characterized in that:

(1) it has affinity for the α1-6 fucose sugar chain represented by a binding constant of $1.0\times10^4$ $M^{-1}$ or more (at 25°C);
(2) it does not substantially bind to the high-mannose sugar chain containing no α1-6 fucose sugar chain and/or the glycolipid sugar chain containing no α1-6 fucose.

In this specification, the binding constant means a value measured at an analysis temperature of 25°C by using, for example, frontal affinity chromatography (FAC method). The FAC method is detailed in Patent Document 1 filed by the present applicant, for example. Patent Document 1 is incorporated in this specification for reference.

The binding constant (at 25°C) of the α1-6 fucose-specific lectin for the (1) α1-6 fucose sugar chain is preferably $5.0\times10^4$ $M^{-1}$ or more, more preferably $1.0\times10^5$ $M^{-1}$ or more, and even more preferably $10\times10^6$ $M^{-1}$ or more.

In this specification, the phrase "(2) it does not substantially bind to the high-mannose sugar chain containing no α1-6 fucose sugar chain and/or the glycolipid sugar chain containing no α1-6 fucose sugar chain." regarding the α1-6 fucose-specific lectin means that the binding constant (at 25°C) for the high-mannose sugar chain containing no α1-6 fucose sugar chain and/or the glycolipid sugar chain containing no α1-6 fucose is typically $10\times10^3$ $M^{-1}$ or less, preferably $10\times10^2$ $M^{-1}$ or less, and particularly preferably 0.

A molecular weight of the α1-6 fucose-specific lectin by SDS electrophoresis is typically 4,000 to 40,000, and preferably 4,000 to 20,000. Herein, the molecular weight by SDS electrophoresis is measured according to a method of Laemmi (Nature, Vol. 227, P. 680, 1976), for example. The α1-6 fucose-specific lectin may be a lectin in which typically 2 to 10, preferably 2 to 6, and more preferably 2 to 3 subunits are bound.

The α1-6 fucose-specific lectin also has a high affinity for an α1-6 fucose sugar chain having a sialic acid at its non-reducing terminal. On the other hand, LCA, NPA and PSA have a low affinity for the α1-6 fucose sugar chain having a sialic acid at the non-reducing terminal.

The α1-6 fucose-specific lectin further has an affinity represented by a binding constant (at 25°C) of preferably $10\times10^4$ $M^{-1}$ or more, more preferably $5.0\times10^4$ $M^{-1}$ or more, and even more preferably $1.0\times10^5$ $M^{-1}$ or more to N-linked single chain, double chains, triple chains and/or quadruple chains bound with the α1-6 fucose.

The α1-6 fucose-specific lectin can be extracted and/or purified from natural products. Methods for obtaining a naturally derived α1-6 fucose-specific lectin are detailed in Patent Document 1 filed by the present applicant and Non-Patent Document 1 posted by the present applicant. Note that the Pholiota terrestris lectin (PTL) described in Patent Document 1 is replaced with Pholiota squarrosa lectin (PhoSL). The α1-6 fucose-specific lectin obtained from natural products will be outlined below.

The above-described natural products are mushrooms such as basidiomycete and ascomycete, for example. Strophariaceae, Tricholomataceae, Polyporaceae and Amanitaceae belong to basidiomycete. Strophariaceae includes Pholiota squarrosa, Pholiota terrestris, Stropharia rugosoannulata, Naematoloma sublateritium, Pholiota aurivella, and Pholiota adiposa. Tricholomataceae includes Lepista sordida. Polyporaceae includes Trichaptum elongatum, and Microporus vernicipes. Amanitaceae includes Amanita muscaria.

Among these basidiomycetes or ascomycetes, Strophariaceae, Tricholomataceae or Amanitaceae are particularly preferable, and Pholiota squarrosa, Pholiota terrestris, Pholiota aurivella, Stropharia rugosoannulata, Naematoloma sublateritium, Lepista sordida or Amanita muscaria are more preferable, from the viewpoints of recognition specificity of lectin for the α1-6 fucose sugar chain and recovery efficiency of lectin.

The α1-6 fucose-specific lectin is preferably a lectin derived from basidiomycete, more preferably Pholiota squarrosa lectin (PhoSL), Pholiota terrestris lectin (PTL), Stropharia rugosoannulata lectin (SRL), Naematoloma sublateritium lectin (NSL), Lepista sordida lectin (LSL) and Amanita muscaria lectin (AML), and even more preferably PhoSL, PTL, SRL, NSL and AML.

The α1-6 fucose-specific lectin can be isolated from basidiomycete or ascomycete by appropriately combining known extraction methods, separation methods, purification methods and the like, for example. For example, a step of obtaining an aqueous medium extract of basidiomycetes and/or ascomycetes using an aqueous medium as an extraction solvent is included. The site for use of these basidiomycetes and/or ascomycetes is preferably a fruit body. From this extract, a lectin is obtained which has a molecular weight by SDS electrophoresis of typically 4,000 to 40,000, and preferably 4,000 to 20,000, and an affinity represented by a binding constant (at 25°C) for the α1-6 fucose sugar chain of typically $10\times10^4$ $M^{-1}$ or more, preferably $5.0\times10^4$ $M^{-1}$ or more, more preferably $1.0\times10^5$ $M^{-1}$ or more, and even more

preferably $10\times10^6$ $M^{-1}$ or more.

Also, the α1-6 fucose-specific lectin may be a peptide or protein chemically synthesized on the basis of an amino acid sequence of a naturally derived lectin, as well as the naturally derived extracts described above. Furthermore, the chemically synthesized peptide or protein may be a peptide (e.g. PhoSL peptide consisting of the amino acid sequence represented by SEQ ID No. 2 appended herein) in which one or a few amino acids in an amino acid sequence of a naturally-derived lectin (e.g. PhoSL consisting of the amino acid sequence represented by SEQ ID No. 1 appended herein) are replaced with lysine and/or arginine and which has a carbohydrate-binding activity. A method for synthesizing them is detailed in Patent Document 4 filed by the present applicant. The specification of Patent Document 4 is incorporated in this specification for reference.

Also, the α1-6 fucose-specific lectin may be a recombinant (e.g. the PhoSL recombinant lectin shown in Example 18) artificially expressed in a known host different from a natural origin by using a nucleic acid encoding an amino acid sequence of a naturally derived lectin, as well as the naturally derived extracts described above. A method for expressing the recombinant is detailed in Patent Document 5. The specification of Patent Document 5 filed by the present applicant is incorporated in this specification for reference.

In the α1-6 fucose-specific lectin, a labeling means which allows detection of the α1-6 fucose-specific lectin is preferably incorporated. For the labeling means, a known labeling method can be applied without any particular limitation, and may include e.g. labeling with a radioisotope, binding with a labeling compound, etc. The radioisotope may include e.g. $^{14}C$, $^{3}H$ and $^{32}P$.

The labeling compound may include e.g. enzyme label (horseradish peroxidase, alkaline phosphatase, etc.), biotin label, digoxigenin label, fluorescent label (fluorescein isothiocyanate, CyDye (registered trademark), ethyl 4-aminobenzoate (ABEE), aminopyridine, allophycocyanin, phycoerythrin, etc.), etc. These labeling compounds can bind to lectins by a conventional method. In particular, the biotin label is preferable in light of high sensitivity.

The present invention also provides a method for enhancing signals based on a reaction between an α1-6 fucose sugar chain and an α1-6 fucose-specific lectin binding thereto, wherein the method includes a step of reacting the sugar chain with the lectin in the presence of a signal enhancer having, as an active ingredient, at least one selected from a group consisting of urea and thiourea.

A concentration of urea in the reaction step may be typically 1 to 9 M, and is preferably 2.5 to 9 M, more preferably 3 to 8 M, and particularly preferably 3.5 to 7 M. In addition, a concentration of thiourea in the reaction step may be typically 0.1 to 1.5 M, and is preferably 0.6 to 1.5 M, more preferably 0.8 to 1.5 M, and particularly preferably 1 to 1.4 M.

The present invention also provides a method for enhancing signals based on a reaction between an α1-6 fucose sugar chain and an α1-6 fucose-specific lectin binding thereto, wherein the method includes a step of washing the sugar chain with a detergent liquid containing a signal enhancer having, as an active ingredient, at least one selected from a group consisting of urea and thiourea, and a step of reacting the sugar chain with the lectin.

A concentration of urea in the detergent liquid may be typically 1 to 9 M, and is preferably 2.5 to 9 M, more preferably 3 to 8 M, and particularly preferably 3.5 to 7 M. In addition, a concentration of thiourea in the detergent liquid may be typically 0.1 to 1.5 M, and is preferably 0.6 to 1.5 M, more preferably 0.8 to 1.5 M, and particularly preferably 1 to 1.4 M. In addition, the step of reacting the sugar chain with the lectin may be carried out in the presence of the signal enhancer having, as an active ingredient, at least one selected from a group consisting of urea and thiourea.

In the above-described method, the means for detecting the α1-6 fucose sugar chain reacted with α1-6 fucose-specific lectin is not particularly limited. As detection means, for example ELISA (direct adsorption method, sandwich method and competitive method), lectin affinity chromatography, lectin staining, lectin chip, flow cytometric (FACS) method, agglutination method, surface plasmon resonance method (e.g. Biacore (registered trademark) system), electrophoresis, beads, etc. can be used. Some representative detection methods will be outlined below.

In the direct adsorption ELISA method, a specimen such as blood is added to a plate and immobilized. Subsequently, a biotin-labeled lectin is added together with a signal enhancer to react a sugar chain with the lectin in the presence of the signal enhancer. Alternatively, a detergent liquid containing the signal enhancer may be added in advance, and after its disposal, a lectin solution containing no signal enhancer may be added. As a secondary labeling compound, an HRP (horseradish peroxidase)-labeled streptavidin solution is added to react biotin with streptavidin. Subsequently, a chromogenic substrate for HRP is added to develop color, and a coloring intensity is measured by an absorptiometer. If a calibration curve is prepared with a standard sample containing a sugar chain at a known concentration in advance, the sugar chain can also be quantified.

In the sandwich ELISA method, an antibody which binds to an antigen having an α1-6 fucose sugar chain is added to a plate and immobilized, and then a specimen such as serum is added. Subsequently, an biotin-labeled lectin is added together with a signal enhancer to react the sugar chain with the lectin in the presence of the signal enhancer. Alternatively, a detergent liquid containing the signal enhancer may be added in advance, and after its disposal, a lectin solution containing no signal enhancer may be added. As a secondary labeling compound, an HRP (horseradish peroxidase)-labeled streptavidin solution is added to react biotin with streptavidin. Subsequently, a chromogenic substrate

for HRP is added to develop color, and a coloring intensity is measured by an absorptiometer. If a calibration curve is prepared with a standard sample at a known concentration in advance, the α1-6 fucose sugar chain can also be quantified.

The lectin affinity chromatography is affinity chromatography utilizing a property of a lectin immobilized on a carrier to specifically bind to a sugar chain. High throughput can be expected by combination with HPLC.

As carriers for immobilizing the lectin, gel materials such as agarose, dextran, cellulose, starch and polyacrylamide are generally used. For them, commercially available products can be used without particular limitation, and may include e.g. sepharose 4B and sepharose 6B (both manufactured by GE Healthcare Bioscience Co., Ltd.). Columns used for lectin chromatography may also include columns with lectin immobilized on a microplate and a nanowell.

The concentration of lectin to be immobilized is typically 0.001 to 100 mg/mL, and preferably 0.01 to 20 mg/mL. When the carrier is an agarose gel, it is activated with CNBr or the like and then coupled with lectin. The lectin may be immobilized on the gel to which the activation spacer has been introduced. Furthermore, the lectin may be immobilized on a gel to which a formyl group has been introduced, and then reduced with $NaCNBH_3$. In addition, a commercially available activated gel such as NHS-Sepharose (manufactured by GE Healthcare Bioscience Co., Ltd.) may be used.

After the specimen is supplied to the column together with the signal enhancer, the column is flushed with a buffer for washing. Alternatively, the specimen is supplied to the column in a state where a signal enhancer is added to the buffer. In one example of the buffer, a molar concentration is typically 5 to 500 mM, and preferably 10 to 500 mM, a pH is typically 4.0 to 10.0, and preferably 6.0 to 9.0, an NaCl content is typically 0 to 0.5 M, and preferably 0.1 to 0.2 M, and a $CaCl_2$, $MgCl_2$ or $MnCl_2$ content is typically 0 to 10 mM, and preferably 0 to 5 mM.

After washing the affinity column, the sugar chain is eluted by using a desorbent such as sodium chloride and a hapten sugar, in a neutral unmodified buffer capable of effectively eluting the sugar chain. This buffer may be the same as above. A concentration of the desorbent is preferably 1 to 500 mM, and particularly preferably 10 to 200 mM. Preferably the elution buffer comprises no signal enhancer.

In the lectin staining, a specimen is thinly cut from a tumor tissue and the slice is stuck to a glass plate. The plate is soaked in a blocking buffer and gently stirred at room temperature. Optionally, endogenous peroxidase inactivation is carried out. The plate is soaked in a biotin-labeled lectin solution (diluted to 2 to 5 μg/mL with the blocking buffer) containing a signal enhancer, and gently stirred at room temperature for 1 hour. Alternatively, a detergent liquid containing the signal enhancer may be added in advance, and after disposal of the detergent liquid, a lectin solution containing no signal enhancer may be added. The plate is soaked in an HRP-labeled avidin solution, and gently stirred at room temperature. The plate is soaked in a chromogenic liquid, and color-developed at room temperature for several minutes. When sufficient color development is confirmed, the plate is washed with water to terminate the reaction. The color-developed tissue is observed with an optical microscope. For the lectin staining, a commercially available lectin staining kit can be used.

In the FACS method, cells (specimen) are suspended in a phosphate buffered saline (PBS). The cells are loosen by strongly taking them in and out of a 2.5 mL syringe with a 21G needle about ten times, and then filtered with a 50 μm mesh. To the cells prepared in such a way, a fluorescently labeled lectin solution is added together with a signal enhancer to bind the cells with the lectin in the presence of the signal enhancer. Alternatively, a detergent liquid containing the signal enhancer may be added in advance, and after disposal of the detergent liquid, a lectin solution containing no signal enhancer may be added. Thereafter, the amount and rate of lectin-bound cells are analyzed by a flow cytometer (e.g. Cytomics FC 500 manufactured by Beckman Coulter, Inc.).

The specimen used for the above-described detection means is not particularly limited. The specimen may include e.g. blood, plasma, serum, tear, saliva, body fluid, milk, urine, culture supernatant of cells, secretion from transgenic animals, etc.

Also, the present invention provides a kit for detecting an α1-6 fucose sugar chain, including: a signal enhancer having, as an active ingredient, at least one selected from a group consisting of urea and thiourea; and an α1-6 fucose-specific lectin.

The detection kit may optionally include general-purpose components for the detection kit, such as various labeling compounds, a buffer, a plate, beads, a reaction terminator, etc. Also, the detection kit preferably includes a reagent for extracting a glycoprotein having the α1-6 fucose sugar chain from a specimen obtained from an organism blood (e.g. antigens such as an anti-haptoglobin antibody and an anti-α fetoprotein antibody, and immunoglobulin-binding proteins such as protein A, protein G and protein L).

The applications of the kit for detecting the α1-6 fucose sugar chain of the present invention are not particularly limited as long as involving detection of the α1-6 fucose sugar chain. Specific examples of the applications will be described below.

Highly accurate detection of AFP-L3 band (band of an α-fetoprotein to which the core fucose was transferred) is useful for early diagnosis of hepatocarcinoma clinically associated with cirrhosis, precise follow-up of hepatocarcinoma, accurate determination of therapeutic effects, early detection of embryonal tumor, an indicator for liver regeneration in fulminant hepatitis, etc. Also, a 5β1 integrin to which the core fucose was transferred is expected as an indicator of diagnosing liver cancer.

In colon cancer with low malignancy or its primary cancers, fucoses including the core fucose increases. On the other hand, in colon cancer with high malignancy or its metastatic cancer, $\alpha$1-6 binding fucose tends to decrease. Thus, the $\alpha$1-6 fucose-specific lectin is made to act on tumor tissues of colon cancer of a person diagnosed with colon cancer in primary screening, and an amount of the $\alpha$1-6 binding fucose is examined, thereby the colon cancer with high malignancy and its metastatic cancer can be discriminated.

Once pancreatic cancer is caused, a fucosylated haptoglobin with a core fucose added to a glycoprotein haptoglobin is produced. This fucosylated haptoglobin increases as the stage of pancreatic cancer progresses, and disappears after removal of the tumor portion of pancreatic cancer. The sensitivity of the detection by the $\alpha$1-6 fucose-specific lectin can be enhanced by combining the signal enhancer of the present invention with the reaction between the $\alpha$1-6 fucose-specific lectin and the fucosylated haptoglobin. In addition, the fucosylated haptoglobin is washed with a detergent liquid having the signal enhancer, and after the disposal of the detergent liquid, a lectin solution containing no signal enhancer may be added. Furthermore, according to an assay using the $\alpha$1-6 fucose-specific lectin and the anti-haptoglobin antibody, pancreatic cancer can be quickly and conveniently detected. Furthermore, it is possible to discriminate between pancreatic cancer and pancreatitis by combining a pancreatic cancer marker CA19-9 antibody and the $\alpha$1-6 fucose-specific lectin. In that case, combination with the signal enhancer of the present invention further facilitates the discrimination.

The detection kit of the present invention is expected for use not only in diagnosis and study for the above-described diseases but also in diagnosis and study for: tumors such as prostate cancer, breast cancer, gastric cancer, small intestine cancer, colorectal cancer, renal cell cancer, small cell lung cancer, non-small cell cancer, uterine cancer, ovarian cancer, thyroid cancer, soft tissue sarcoma, osteosarcoma, melanoma, glioblastoma, astrocytoma, medulloblastoma, acute lymphoma, malignant lymphoma, Hodgkin's disease, non-Hodgkin's disease, acute myelogenous leukemia, chronic lymphocytic leukemia; allergic diseases; autoimmune diseases; and cardiovascular diseases such as emphysema.

## Examples

Hereinafter, the present invention will be described in more detail with reference to Examples of the present invention. However, the present invention is not limited to the following Examples.

The reagents used in the present invention were prepared by the following procedure.

(1) Phosphate buffered saline (PBS)

5.75 g of disodium hydrogen phosphate (manufactured by Wako Pure Chemical Industries, Ltd.), 1.0 g of potassium dihydrogenphosphate (manufactured by Wako Pure Chemical Industries, Ltd.), 1.0 g of potassium chloride (manufactured by Wako Pure Chemical Industries, Ltd.) and 40.0 g of sodium chloride (manufactured by Wako Pure Chemical Industries, Ltd.) were weighed out, to which 400 mL of water was added and dissolved, then transferred to a measuring cylinder, and the volume was adjusted to 500 mL with water to prepare a 10-time concentrated phosphate buffered saline (10×PBS). The 10×PBS was diluted by 10 times with water to prepare PBS.

(2) 1% bovine serum albumin (BSA)/PBS

1 g of BSA (manufactured by Wako Pure Chemical Industries, Ltd.) was weighed out and dissolved in 100 mL of PBS to prepare 1% BSA/PBS.

(3) 0.05% Tween/PBS

2.5 mL of polyoxyethylene sorbitan monolaurate (Tween 20, manufactured by Nacalai tesque, inc.) was added to 5 L of PBS.

(4) Biotin-labeled PhoSL

As the $\alpha$1-6 fucose-specific lectin, Pholiota squarrosa lectin (PhoSL, SEQ ID No. 1) was purified from Pholiota squarrosa according to the method described in Non-Patent Document 1. This Pholiota squarrosa lectin was weighed out, to which a 0.1 M sodium bicarbonate solution was added and dissolved (concentration: 5 mg/mL). A biotinylating reagent (model number: B 2643, manufactured by Sigma-Aldrich Co. LLC) was dissolved in dimethylsulfoxide, which was added to the lectin solution and reacted. The reaction liquid was subjected to solvent displacement with water by ultrafiltration (3 K membrane, manufactured by Millipore Corporation), and lyophilized to obtain a biotin-labeled PhoSL.

(5) Biotin-labeled PhoSL peptide

A PhoSL peptide (SEQ ID No. 2) was synthesized based on the description of Example 6 of Patent Document 4 (with the proviso that the PTL in Patent Document 4 was replaced with PhoSL). This lectin was labeled with biotin by the same procedure as for the biotin-labeled PhoSL.

(6) Biotin-labeled SRL

The SRL (SEQ ID No. 3) was extracted according to the method described in Patent Document 2. This lectin was labeled with biotin by the same procedure as for the biotin-labeled PhoSL.

(7) Biotin-labeled Aleuria aurantia lectin (biotin-labeled AAL)

A biotin-labeled Aleuria aurantia lectin (manufactured by J-OIL MILLS, Inc.) was prepared.
The sequence list of the α1-6 fucose-specific lectins (before labeling) in (4) to (6) is shown in Table 1.

[Table 1]

| Name | Amino acid sequence | SEQ ID No. |
|---|---|---|
| PhoSL | APVPVTKLVX DGDTYKXTAX LDXGDGXWVA QTXTXVFHXG | 1 |
| PhoSL peptide | KPVPVTKLVC DGDTYKCTAK LDFGDGRWVA QWDTNVFHK | 2 |
| SRL | APVXVYXLXX DGXSTKXTAX LDYGDGXWXA QWXXNVFHX | 3 |

Xs at positions 10 and 17 in SEQ ID No. 1 may be any amino acid residue, but are preferably Cys, and Xs at positions 20, 23, 27, 33, 35 and 39 are Tyr/Ser, Phe/Tyr, Arg/Lys/Asn, Asp/Gly/Ser, Asn/Ala and Thr/Gln respectively.
SEQ ID No. 2 is a sequence that Ala at position 1, Tyr at position 20 and Thr at position 39 are substituted with Lys, and furthermore Gly at position 40 is deleted in the specific example of SEQ ID No. 1 (APVPVTKLVC DGDTYKCTAY LDFGDGRWVA QWDTNVFHTG).
Xs at position 10 and 7 in SEQ ID No. 3 may be any amino acid residue, but are preferably Cys, and Xs at positions 4, 7, 9, 13, 20, 27, 29, 33, 34 and 39 are Pro/Gly, Glu/Lys, Val/Asp, Asn/Asp/Glu, His/Ser, Lys/His, Val/Ile, Gly/Asn/Ser, Ala/Thr and Arg/Thr, respectively.

[Examples 1 to 3] Signal enhancement test (1)

Glycoproteins having the α1-6 fucose sugar chain were reacted with the α1-6 fucose-specific lectins in the presence or absence of urea by a direct adsorption ELISA method, and the presence of enhanced signals based on the binding reaction between the sugar chain and the lectin was investigated. The specific procedure is as follows.
As glycoproteins having the α1-6 fucose sugar chain, a fucosylated haptoglobin (hereinafter referred to as "fHP"), an α-fetoprotein L3 (hereinafter referred to as "AFP-L3"), and a mouse immunoglobulin G (manufactured by Sigma-Aldrich Co. LLC, hereinafter referred to as "IgG") were prepared. For comparison, a transferrin (manufactured by Sigma-Aldrich Co. LLC, hereinafter referred to as "TF") was also prepared as a glycoprotein other than the α1-6 fucose sugar chain. Methods for preparing fHP and AFP-L3 are as follows.

(1) Preparation of fHP solution

A pancreatic cancer cell line (PSN-1, obtained from DS Pharma Biomedical Co.,Ltd.) was cultured according to a conventional method to obtain a culture supernatant. 1000 mL of the culture supernatant was concentrated to 1 mL with an ultrafiltration filter (product name: VIVA SPIN 20-10K, manufactured by Sartorius AG). The concentrate was added to 0.5 mL of a gel prepared by immobilizing an anti-haptoglobin antibody (manufactured by The Binding Site Ltd.) on NHS-activated Sepharose 4 Fast Flow (manufactured by GE Healthcare Bioscience Co., Ltd.). They were mixed every 10 minutes at room temperature, and after one hour, the solution comprising the gel was added to a 0.45 μm filter tube (manufactured by Millipore Corporation), centrifuged at 400×g, 4°C for 5 minutes, and the filtrate was discarded. Subsequently, 200 μL of PBS was added, the mixture was centrifuged at 400×g, 4°C for 5 minutes, and the filtrate was discarded. This procedure was repeated twice. Subsequently, 200 μL of elution buffer (100 mM glycine, 0.5 M NaCl, pH 3.0) was added, the mixture was centrifuged at 400×g, 4°C for 5 minutes, and the filtrate was collected. This procedure was repeated twice. A solution obtained by combining these solutions was neutralized with 3 N of NaOH, and then 600

μL of PBS was added to obtain an fHP solution. This fHP solution was further diluted with PBS to prepare a 1000 ng/mL fHP solution.

(2) Preparation of AFP-L3 solution

A liver cancer cell line (HepG2, obtained from Institute of Physical and Chemical Research) was cultured according to a conventional method to obtain a culture supernatant. 1000 mL of the culture supernatant was concentrated to 1 mL with the ultrafiltration filter (product name: VIVA SPIN 20-10k, manufactured by Sartorius AG). The concentrate was added to 0.5 mL of a gel prepared by immobilizing an anti-AFP antibody (manufactured by DAKO Inc.) on NHS-activated Sepharose 4 Fast Flow (manufactured by GE Healthcare Bioscience Co., Ltd.). They were mixed every 10 minutes at room temperature, and after one hour, the solution comprising the gel was added to a 0.45 μm filter tube (manufactured by Millipore Corporation), centrifuged at 400×g, 4°C for 5 minutes, and the filtrate was discarded. Subsequently, 200 μL of PBS was added, the mixture was centrifuged at 400×g, 4°C for 5 minutes, and the filtrate was discarded. This procedure was repeated twice. Subsequently, 200 μL of elution buffer (100 mM glycine, 0.5 M NaCl, pH 3.0) was added, the mixture was centrifuged at 400×g, 4°C for 5 minutes, and the filtrate was collected. This procedure was repeated twice. A solution obtained by combining these solutions was neutralized with 3 N NaOH, and then 600 μL of PBS was added to obtain an AFP-L3 solution. This AFP-L3 solution was further diluted with PBS to prepare a 1000 ng/mL AFP-L3 solution.

(3) Preparation of IgG and TF solutions

IgG and TF were dissolved in PBS to prepare a 5000 ng/mL IgG solution and a 5000 ng/mL TF solution, respectively.

The procedure of the direct adsorption ELISA method is shown below.

(1) Immobilization of antigen

50 μL of the above-described solution of fHP, AFP-L 3, IgG or TF was added to each well of a microtiter plate (manufactured by Greiner Bio-One GmbH), left at 4°C for 16 hours, and then the added solution was discarded.

(2) Washing

250 μL of 0.05% Tween/PBS was added to each well, and the added solution was discarded.

(3) Blocking

200 μL of 1% BSA/PBS was added to each well, left at 37°C for 1 hour, and then the added solution was discarded.

(4) Washing

250 μL of 0.05% Tween/PBS was added to each well, and the added solution was discarded. This manipulation was repeated a total of three times.

(5) Lectin reaction

A biotin-labeled PhoSL was diluted with 1% BSA/PBS, and urea (manufactured by Wako Pure Chemical Industries, Ltd.) was added to prepare a lectin solution (the final concentration of the biotin-labeled PhoSL: 1 μg/mL, and the final concentration of urea: 5 M). 50 μL of this lectin solution was added to each well, left at 4°C for 30 minutes, and then the added solution was discarded. As a control, a lectin solution containing no urea (the final concentration of the biotin-labeled PhoSL: 1 μg/mL, and urea: 0 M) was subjected to the same reaction.

(6) Washing

250 μL of 0.05% Tween/PBS was added to each well, and the added solution was discarded. This manipulation was repeated a total of three times.

(7) HRP-labeled streptavidin reaction

50 μL of an HRP-labeled streptavidin solution (manufactured by VECTOR LABORATORIES, INC., prepared to be 1 μg/mL in PBS) was added to each well, left at room temperature for 30 minutes, and then the added solution was discarded.

(8) Washing

250 μL of 0.05% Tween/PBS was added to each well, and the added solution was discarded. This manipulation was repeated a total of three times.

(9) Chromogenic reaction

50 μL of a chromogenic substrate for HRP (product name: TMB Peroxidase substrate system, manufactured by Kirkegaard & Perry Laboratories, Inc.) was added to each well, and left at room temperature for 5 minutes.

(10) Termination of reaction

The reaction was terminated by adding 50 μL of 1 M phosphoric acid.

Absorbances at wavelengths of 450 nm and 630 nm were measured using a plate reader (product name: POWERSCAN (registered trademark) HT, manufactured by DS Pharma Biomedical Co.,Ltd.). A value obtained by subtracting the absorbance value at 630 nm from the absorbance value at 450 nm was taken as a detected value (signal: S). Similarly, a detected value (noise: N) for a well to which no glycoprotein was added was measured, and a value obtained by subtracting the detected value (N) for the well itself from the detected value (S) was taken as a reaction value $(S - N)_{urea\ 5M}$. Also, a reaction value $(S - N)_{urrea\ 0M}$ in the case that the lectin was reacted with the glycoprotein in the absence of urea was determined.

The increased amount and the increased rate of the signals were determined by the following equations respectively:

[Eq. 1]

$$\text{Increased amount of the signal} = (S - N)_{urea\ 5M} - (S - N)_{urea\ 0M}$$

[Eq. 2]

$$\text{Increased rate of the signal} = (S - N)_{urea\ 5M} / (S - N)_{urea\ 0M}$$

The reaction values (S - N) of PhoSL with respect to various glycoproteins are shown in Table 2 and Figure 1.

[Table 2]

| | Lectin | Glycoprotein | Reaction value | | | |
|---|---|---|---|---|---|---|
| | | | $(S-N)_{urea\ 0M}$ | $(S-N)_{urea\ 5M}$ | Increased amount of signal | Increased rate of signal |
| Example 1 | PhoSL | fHP | 0.253 | 0.724 | 0.471 | 2.86 |
| Example 2 | | AFP-L3 | 0.008 | 0.132 | 0.124 | 16.5 |
| Example 3 | | IgG | 0.625 | 0.878 | 0.253 | 1.40 |
| Comp. Example 1 | | TF | 0.006 | 0.011 | 0.005 | 1.83 |

$(S - N)_{urea\ 0M}$: Reaction value (S - N) when reacted in the absence of urea
$(S - N)_{urea\ 5M}$: Reaction value (S - N) when reacted in the presence of 5 M urea

Table 2 and Figure 1 show that both the increased amount and the increased rate of the signals based on the

reaction between fHP, AFP-L3 or IgG and PhoSL in the presence of urea were significantly increased compared to the case without urea. On the other hand, the signals based on the reaction between TF other than the α1-6 fucose sugar chain and PhoSL showed a high increased rate, but the increased amount was not significantly increased.

[Examples 4 to 6] Signal enhancement test (2)

fHP as a glycoprotein having the α1-6 fucose sugar chain was reacted with various α1-6 fucose-specific lectins in the presence or absence of urea by a sandwich ELISA method, and the presence of enhanced signals based on the binding reaction between the glycoprotein and the lectin was investigated. The specific procedure is as follows.

As the α1-6 fucose-specific lectin, a biotin-labeled PhoSL, a biotin-labeled PhoSL peptide, and a biotin-labeled SRL were used. For comparison, a biotin-labeled AAL which also binds to a sugar chain having a fucose other than the α1-6 fucose was used.

The procedure of the sandwich ELISA method is shown below.

(1) Immobilization of antigen

An anti-haptoglobin antibody from which a sugar chain was removed (manufactured by NIPPON BIO-TEST LABORATORIES INC.) was diluted to 5 μg/mL with PBS, 50 μL of the diluent was added to each well of a microtiter plate (manufactured by Greiner Bio-One GmbH), left at 4°C for 16 hours, and then the added solution was discarded.

(2) Washing

250 μL of 0.05% Tween/PBS was added to each well, and the added solution was discarded.

(3) Blocking

200 μL of 1% BSA/PBS was added to each well, left at 37°C for 1 hour, and then the added solution was discarded.

(4) Washing

250 μL of 0.05% Tween/PBS was added to each well, and the added solution was discarded. This manipulation was repeated a total of three times.

(5) Antigen-antibody reaction

50 μL of 200 ng/mL fHP diluted with 1% BSA/PBS was added to each well, left at room temperature for 1 hour, and then the added solution was discarded.

(6) Washing

250 μL of 0.05% Tween/PBS was added to each well, and the added solution was discarded. This manipulation was repeated a total of three times.

(7) Lectin reaction

A biotin-labeled PhoSL, a biotin-labeled PhoSL peptide, a biotin-labeled SRL or a biotin-labeled AAL was diluted with a 1% BSA/PBS solution, and urea (manufactured by Wako Pure Chemical Industries, Ltd.) was added to prepare each lectin solution (the final concentration of the biotin-labeled lectin: 1 μg/mL, and the final concentration of urea: 5 M). 50 μL of this lectin solution was added to a well, left at 4°C for 30 minutes, and then the added solution was discarded. As a control, a lectin solution containing no urea (the final concentration of the biotin-labeled lectin: 1 μg/mL, and urea: 0 M) was subjected to the same reaction.

(8) Washing

250 μL of 0.05% Tween/PBS was added to each well, and the added solution was discarded. This manipulation was repeated a total of three times.

(9) HRP-labeled streptavidin reaction

50 μL of an HRP-labeled streptavidin solution (1 μg/mL) was added to each well, left at room temperature for 30 minutes, and then the added solution was discarded.

(10) Washing

250 μL of 0.05% Tween/PBS was added to each well, and the added solution was discarded. This manipulation was repeated a total of three times.

(11) Chromogenic reaction

50 μL of a chromogenic substrate for HRP (product name: TMB Peroxidase substrate system, manufactured by Kirkegaard & Perry Laboratories, Inc.) was added to each well, and left at room temperature for 5 minutes.

(12) Termination of reaction

The reaction was terminated by adding 50 μL of 1 M phosphoric acid.

Absorbances at wavelengths of 450 nm and 630 nm were measured using the plate reader in the same way as Example 1, and furthermore a reaction value $(S - N)_{urea\ 5M}$ was determined. Also, a reaction value $(S - N)_{urea\ 0M}$ in the case that the lectin was reacted with the glycoprotein in the absence of urea was determined. The reaction values (S-N) in the presence and absence of urea, and their increased amounts and increased rates were shown in Table 3 and Figure 2.

[Table 3]

| | Lectin | Glycoprotein | Reaction value | | | |
|---|---|---|---|---|---|---|
| | | | $(S-N)_{urea\ 0M}$ | $(S-N)_{urrea\ 5M}$ | Increased amount of signal | Increased rate of signal |
| Example 4 | PhoSL | fHP | 0.265 | 0.796 | 0.531 | 3.00 |
| Example 5 | PhoSL peptide | | 0.162 | 0.214 | 0.052 | 1.32 |
| Example 6 | SRL | | 0.430 | 0.863 | 0.433 | 2.01 |
| Comp. Example 2 | AAL | | 0.877 | 0.774 | -0.103 | 0.88 |
| $(S - N)_{urea\ 0M}$: Reaction value (S - N) when reacted in the absence of urea<br>$(S - N)_{urea\ 5M}$: Reaction value (S - N) when reacted in the presence of 5 M urea | | | | | | |

Figure 2 and Table 3 show that, in the reaction between PhoSL, PhoSL peptide or SRL and fHP in the presence of 5 M urea (final concentration), both the increased amount and the increased rate of the signals were significantly improved compared to the case without urea. On the other hand, the AAL also binding to the fucose other than the α1-6 fucose did not enhance the signals even by adding urea to the reaction system.

As indicated in Examples 1 to 6, the signal enhancer of the present invention enhances the signals based on the reaction between the α1-6 fucose sugar chain and the α1-6 fucose-specific lectin binding thereto. Urea does not sensitize the reaction between the transferrin having no α1-6 fucose sugar chain and the α1-6 fucose-specific lectin (Comparative Example 1). Urea also does not sensitize the reaction between the sugar chain having the α1-6 fucose sugar chain and the AAL having affinity for the α1-6 fucose but no specificity thereto (Comparative Example 2). Although data is not shown, urea did not sensitize the reaction between the α1-6 fucose sugar chain and the UEA-1, Lotus, ConA, or the like having no affinity for the α1-6 fucose. From the above description, it can be said that the signal enhancement by the signal enhancer of the present invention is a phenomenon occurring only in the reaction between the α1-6 fucose sugar chain and the α1-6 fucose-specific lectin.

[Examples 7 and 8] Inhibition test for signal enhancing action

In order to investigate whether the signal enhancing effect of the signal enhancer of the present invention results

from the specific bond between the α1-6 fucose sugar chain and the α1-6 fucose-specific lectin, an inhibition test using a sandwich ELISA method was carried out.

In Example 7, fucose (manufactured by Nacalai tesque, Inc.) at a final concentration of 20 mM or 100 mM was made to coexist in the reaction between fHP and PhoSL in the presence of 5 M urea. In Example 8, instead of the fucose of Example 7, thyroglobulin (manufactured by Sigma-Aldrich Co. LLC, hereinafter referred to as "TG") at a final concentration of 0.01 mg/mL or 0.1 mg/mL was made to coexist. It is known that the sugar chain-specific bond between fHP and PhoSL is inhibited when a monosaccharide fucose is made to coexist at about 20 to 100 mM. Also, it is known that the TG having the α1-6 fucose sugar chain similarly inhibits the sugar chain-specific bond between fHP and PhoSL.

Absorbances at wavelengths of 450 nm and 630 nm were measured using the plate reader in the same way as in Example 1, and furthermore the reaction value (S - N) was determined. The detection results when adding the fucose or TG are shown in Figure 3.

Figure 3 shows that the reaction value (S - N) decreases as the concentration of the added fucose or TG increases. Based on that, it was confirmed that the effect of increasing the signals based on the bond between the lectin and the glycoprotein in the presence of urea resulted from the specific bond between the α1-6 fucose-specific lectin and the α1-6 fucose sugar chain.

[Example 9] Test of changing the urea concentration

A suitable concentration range of urea as a signal enhancer was examined by a sandwich ELISA method. The same procedure as in Example 4 was carried out except that fHP (50 ng/mL) was used and the final urea concentration relative to the lectin solution was adjusted to 0 to 9 M.

In the same way as Example 1, the absorbances at wavelengths of 450 nm and 630 nm were measured using the plate reader, and furthermore the reaction value (S - N) was determined. The increased rate of the signals at each final concentration of urea compared to the case without urea is shown in Figure 4.

Figure 4 shows that the signals are enhanced by 1.2 to 2.8 times in the presence of urea at a final concentration of 1 to 9 M. The final concentration of urea is preferably 3 to 9 M, more preferably 3 to 8 M, and particularly preferably 3 to 7 M.

[Examples 10 and 11] Test for thiourea

In a direct adsorption ELISA method, a glycoprotein (fHP or AFP-L3) having the α1-6 fucose sugar chain was reacted with the α1-6 fucose-specific lectin (PhoSL) in the presence or absence of thiourea to investigate the presence of the enhanced signals based on the binding reaction between the sugar chain and the lectin. The procedure was the same as in Example 1 except that 5 M urea was replaced by 1.2 M thiourea (manufactured by Wako Pure Chemical Industries, Ltd.).

The reaction value (S - N) was determined from the obtained absorbance in the same way as Example 1. Also, the reaction value (S - N) when reacting the lectin with the glycoprotein in the absence of thiourea was determined.

The increased amount and the increased rate of the signals were determined by the following equations respectively:

[Eq. 3]

$$\text{The increased amount of the signals} = (S - N)_{\text{thiourea 1.2M}} - (S - N)_{\text{thiourea 0M}}$$

[Eq. 4]

$$\text{The increased rate of the signals} = (S - N)_{\text{thiourea 1.2M}} / (S - N)_{\text{thiourea 0M}}$$

The reaction values (S-N) of PhoSL with respect to various glycoproteins are shown in Table 4.

[Table 4]

| | Lectin | Glycoprotein | Reaction value | | | |
|---|---|---|---|---|---|---|
| | | | $(S-N)_{thiourea\ 0M}$ | $(S-N)_{thiourea\ 1.2M}$ | Increased amount of signal | Increased rate of signal |
| Example 10 | PhoSL | fHP | 0.360 | 1.041 | 0.681 | 2.89 |
| Example 11 | | AFP-L3 | 0.003 | 0.117 | 0.114 | 39.0 |
| $(S - N)_{thiourea\ 0M}$: Reaction value (S - N) when reacted in the absence of thiourea<br>$(S - N)_{thiourea\ 1.2M}$: Reaction value (S - N) when reacted in the presence of 1.2M thiourea | | | | | | |

In relation to the signals based on the reaction of fHP and AFP-L3 with PhoSL in the presence of thiourea, both the increased amount and the increased rate of the signals were significantly increased compared to the case without thiourea.

[Example 12] Test of changing the thiourea concentration

A suitable concentration range of thiourea as a signal enhancer was examined by a sandwich ELISA method. The same procedure as in Example 4 was carried out except that the thiourea was used instead of urea and the final thiourea concentration relative to the lectin solution was adjusted to 0 to 1.5 M.

In the same way as Example 1, the absorbances at wavelengths of 450 nm and 630 nm were measured using the plate reader, and furthermore the reaction value (S - N) was determined. In the same way as Example 10, the increased rate of the signals at each final concentration of thiourea compared to the case without thiourea was determined and the result is shown in Figure 5.

Figure 5 shows that the signals are enhanced by 1.2 to 4.1 times in the presence of thiourea at a final concentration of 0.1 to 1.5 M. The final concentration of thiourea may be typically 0.1 to 1.5 M, preferably 0.6 to 1.5 M, more preferably 0.8 to 1.5 M, and particularly preferably 1 to 1.4 M.

[Examples 13 and 14] Test of adding signal enhancer to detergent liquid

In a sandwich ELISA method, a glycoprotein (fHP) comprising the $\alpha$1-6 fucose sugar chain was added, then washed with a solution comprising urea or thiourea, the solution was discarded, and then the glycoprotein was reacted with a lectin solution to investigate the presence of the enhanced signals based on the binding reaction between the sugar chain and the lectin.

Specifically, the same procedure as in Example 4 was carried out except that urea at a final concentration of 5 M or thiourea at a final concentration 1.2 M was added to a detergent liquid (0.05% Tween/PBS) in "(6) Washing" and that urea was not added to the lectin solution in "(7) Lection reaction".

Absorbances at wavelengths of 450 nm and 630 nm were measured using the plate reader in the same way as Example 1, and furthermore a reaction value (S - N) was determined. In addition, the increased amounts and the increased rates of the signals in the case that urea or thiourea was added to the detergent liquid compared to the case without urea and thiourea were calculated in the same way as described above, and shown in Table 5.

[Table 5]

| | Concentration of urea or thiourea in detergent liquid | Glycoprotein | Reaction value | | | |
|---|---|---|---|---|---|---|
| | | | $(S-N)_{0M}$ | $(S-N)_{urea\ 5M}$ or $(S-N)_{thiourea\ 1.2M}$ | Increased amount of signal | Increased rate of signal |

(continued)

| | Concentration of urea or thiourea in detergent liquid | Glycoprotein | Reaction value | | | |
|---|---|---|---|---|---|---|
| | | | $(S\text{-}N)_{0M}$ | $(S\text{-}N)_{urea\ 5M}$ or $(S\text{-}N)_{thiourea\ 1.2M}$ | Increased amount of signal | Increased rate of signal |
| Example 13 | urea 5M | fHP | 0.545 | 1.002 | 0.457 | 1.84 |
| Example 14 | thiourea 1.2M | | 0.545 | 0.909 | 0.364 | 1.67 |
| $(S - N)_{0M}$: Reaction value (S - N) when reacted in the absence of urea and thiourea<br>$(S - N)_{urea\ 5M}$: Reaction value (S - N) when reacted in the presence of 5 M urea<br>$(S - N)_{thiourea\ 1.2M}$: Reaction value (S - N) when reacted in the presence of 1.2 M thiourea | | | | | | |

Table 5 demonstrates that the signals based on the binding reaction between the sugar chain and the lectin can also be enhanced when the α1-6 fucose-specific lectin is reacted after washing a glycoprotein having the α1-6 fucose sugar chain with a detergent liquid containing urea or thiourea.

[Example 15 to 17]

Like Pholiota squarrosa lectin, Pholiota terrestris lectin (PTL), Naematoloma sublateritium lectin (NSL) and Amanita muscaria lectin (AML) were purified from Pholiota terrestris, Naematoloma sublateritium and Amanita muscaria. The obtained lectins were labeled with biotin by the same procedure as for the biotin-labeled PhoSL to obtain a biotin-labeled PTL, a biotin-labeled NSL and a biotin-labeled AML.

The sandwich ELISA was carried out in the same way as Example 4 except that the biotin-labeled PhoSL was replaced with the biotin-labeled PTL, the biotin-labeled NSL or the biotin-labeled AML. The results are shown in Table 6.

[Table 6]

| | Lectin | Glycoprotein | Reaction value | | | |
|---|---|---|---|---|---|---|
| | | | $(S\text{-}N)_{urea\ 0M}$ | $(S\text{-}N)_{urea\ 5M}$ | Increased amount of signal | Increased rate of signal |
| Example 15 | PTL | fHP | 0.542 | 1.285 | 0.743 | 2.37 |
| Example 16 | NSL | | 0.423 | 1.345 | 0.922 | 3.18 |
| Example 17 | AML | | 0.027 | 0.548 | 0.521 | 20.3 |
| $(S - N)_{urea\ 0M}$: Reaction value (S - N) when reacted in the absence of urea<br>$(S - N)_{urea\ 5M}$: Reaction value (S - N) when reacted in the presence of 5 M urea | | | | | | |

As shown in Table 6, signals were proved to be enhanced in the presence of urea in all α1-6 fucose-specific lectins.

[Example 18] Detection method using beads

According to an instruction of magnetic beads (14204, manufactured by Invitrogen Corporation), antibody-immobilizing magnetic beads were prepared by immobilizing the anti-haptoglobin antibody from which the sugar chains had been removed.

Detection using beads was carried out according to a conventional method. Specifically, a fucosylated haptoglobin (fHP) (final concentration: 0 μg/mL or 0.45 μg/mL), urea (final concentration 0 M or 5 M), a PhoSL recombinant lectin shown in SEQ No. 4 (SEQ No. 1 of Patent Document 5: JP 2011-148735 A with an added DYKDDDDK tag, final concentration 5 μg/mL) were mixed to form a complex. The solution containing the complex was diluted by 10 times,

the antibody-immobilizing magnetic beads were added, the solution was washed with a magnet, and then an HRP-labeled anti-FLAG antibody (manufactured by Sigma-Aldrich Co. LLC) was added to produce a complex with the detected antibody. The complex was color-developed using a chromogenic substrate for the HRP, and the chromogenic reaction was terminated using 1 M phosphoric acid. The absorbance of the obtained solution was measured with the plate reader in the same way as described above.

A value obtained by subtracting the absorbance value at 630 nm from the absorbance value at 450 nm in the presence of fHP was taken as a detected value (signal: S), and a value obtained in the same way in the absence of fHP was taken as a detected value (noise: N). A value obtained by subtracting the detected value (N) in the well itself from the detected value (S) in the presence of 5 M urea was taken as a reaction value $(S - N)_{urea\ 5M}$. In addition, a reaction value $(S - N)_{urea\ 0M}$ in the absence of urea was determined in the same way. In addition, a value obtained by dividing the detected value S in the presence of 5 M urea by the detected value N was taken as a reaction value $(S/N)_{urea\ 5M}$. A value obtained in the same way in the absence of urea was taken as a reaction value $(S/N)_{urea\ 0M}$. In the same way as Example 1, the increased amount and the increased rate of the signals were calculated. The results are shown in Table 7.

[Table 7]

| | Lectin | Glycoprotein | Reaction value | | | | | |
|---|---|---|---|---|---|---|---|---|
| | | | $(S\text{-}N)_{urea\ 0M}$ | $(S\text{-}N)_{urea\ 5M}$ | Increased amount of signal | Increased rate of signal | $(S/N)_{urea\ 0M}$ | $(S/N)_{urea\ 5M}$ |
| Example 18 | Recombinant lectin | fHP | 0.455 | 0.708 | 0.253 | 1.56 | 6.83 | 26.29 |

$(S - N)_{urea\ 0M}$: Reaction value (S - N) when reacted in the absence of urea
$(S - N)_{urea\ 5M}$: Reaction value (S - N) when reacted in the presence of 5 M urea
$(S/N)_{urea\ 0M}$: Reaction value (S/N)when reacted in the absence of urea
$(S/N)_{urea\ 5M}$: Reaction value (S/N)when reacted in the presence of 5 M urea

Figure 7 shows that, in relation to the signals based on the reaction between fHP and the recombinant lectin in the presence of urea, both the increased amount and the increased rate of the signals were significantly increased compared to the case without thiourea. Similarly, the reaction value (S/N)also remarkably increases compared to the case without urea. The detection method using the beads could also confirm that the effects of the present invention could be obtained.

## Claims

1. A signal enhancer which enhances signals based on a reaction between an α1-6 fucose sugar chain and an α1-6 fucose-specific lectin binding to the α1-6 fucose sugar chain, wherein the signal enhancer has, as an active ingredient, at least one selected from a group consisting of urea and thiourea.

2. The method according to claim 1, wherein a concentration of urea in the signal enhancer is 1 to 9 M.

3. The method according to claim 1, wherein a concentration of thiourea in the signal enhancer is 0.1 to 1.5 M.

4. A method for enhancing signals based on a reaction between an α1-6 fucose sugar chain and an α1-6 fucose-specific lectin binding to the α1-6 fucose sugar chain, wherein the method includes a step of reacting the sugar chain with the lectin in the presence of a signal enhancer having, as an active ingredient, at least one selected from a group consisting of urea and thiourea.

5. The method according to claim 4, wherein a concentration of the urea in the reaction is 1 to 9 M.

6. The method according to claim 4, wherein a concentration of the thiourea in the reaction is 0.1 to 1.5 M.

7. A method for enhancing signals based on a reaction between an α1-6 fucose sugar chain and an α1-6 fucose-specific lectin binding to the α1-6 fucose sugar chain, wherein
the method includes: a step of washing the sugar chain with a detergent liquid which contains a signal enhancer having, as an active ingredient, at least one selected from a group consisting of urea and thiourea; and
a step of reacting the sugar chain with the lectin.

8. The method according to claim 7, wherein the step of washing is performed immediately before the step of reacting.

9. The method according to claim 7, wherein a concentration of urea in the detergent liquid is 1 to 9 M.

10. The method according to claim 7, wherein a concentration of thiourea in the detergent is 0.1 to 1.5 M.

11. A kit for detecting an α1-6 fucose sugar chain, wherein the kit comprises:

    a signal enhancer having, as an active ingredient, at least one selected from a group consisting of urea and thiourea; and
    an α1-6 fucose-specific lectin.

1.0
0.8
0.6
0.4
0.2
0.0
Reaction value (S-N)
Urea 0M
Urea 5M
1000ng/ml fHP
1000ng/ml AFP-L3
5000ng/ml IgG
5000ng/ml TF
Example 1
Example 2
Example 3
Comp.Example 1

Fig.1

1.0
0.8
0.6
0.4
0.2
0.0
Reaction value (S-N)
Urea 0M
Urea 5M
PhoSL
PhoSL
SRL
AAL
Example 4
Example 5
Example 6
Comp.Example 2

Fig.2

1
0.8
0.6
0.4
0.2
0
Reaction value (S-N)
0 mM
20 mM
200 mM
0 mg/ml
0.1 mg/ml
1 mg/ml
Concentration of fucose
Concentration of thyroglobulin
Example 7
Example 8

Fig.3

Increased rate of signal
0
0.5
1
1.5
2
2.5
3
0
1
2
3
4
5
6
7
8
9
10
Concentration of urea (M)

Fig.4

Increased rate of signal
0.0
0.5
1.0
1.5
2.0
2.5
3.0
3.5
4.0
4.5
0
0.2
0.4
0.6
0.8
1
1.2
1.4
1.6
Concentration of thiourea (M)

Fig.6

## INTERNATIONAL SEARCH REPORT

International application No.
PCT/JP2016/055541

A. CLASSIFICATION OF SUBJECT MATTER
*G01N33/574*(2006.01)i

According to International Patent Classification (IPC) or to both national classification and IPC

B. FIELDS SEARCHED

Minimum documentation searched (classification system followed by classification symbols)
G01N33/574

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2016 |
| Kokai Jitsuyo Shinan Koho | 1971-2016 | Toroku Jitsuyo Shinan Koho | 1994-2016 |

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| A | JP 2010-145202 A (Tosoh Corp.), 01 July 2010 (01.07.2010), (Family: none) | 1-11 |
| A | WO 2011/089988 A1 (J-Oil Mills, Inc.), 28 July 2011 (28.07.2011), & JP 4900983 B2 & US 8481275 B2 & EP 2498094 A1 | 1-11 |
| A | Yuka KOBAYASHI, "Screening for New Lectins and Their Applications", Oyo Toshitsu Kagaku, 2013, vol.3, no.3, pages 200 to 201 | 1-11 |
| A | WO 2014/046248 A1 (Mitsubishi Chemical Corp.), 27 March 2014 (27.03.2014), (Family: none) | 1-11 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 13 May 2016 (13.05.16) | 24 May 2016 (24.05.16) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4514163 B
- JP 5263979 B
- JP 4900983 B
- JP 2011148736 A
- JP 2011148735 A
- JP 2010145202 A


### Non-patent literature cited in the description


- **YUKA KOBAYASHI et al.** A Novel Core Fucose-specific Lectin from the Mushroom Pholiota squarrosa. *J. Biol. Chem,* 2012, vol. 287, 33973-33982
- **LAEMMI.** *Nature,* 1976, vol. 227, 680